# EUROPEAN PATENT APPLICATION

(11) **EP 4 574 041 A1**
(43) Date of publication of application: **25.06.2025**
(21) Application number: 24170159.8
(22) Date of filing: 15.04.2024
(51) Int. Cl.: A61B 5/16, A61B 5/00, G06V 40/16, A61B 5/01

(54) **ELECTRONIC SYSTEM AND PHYSIOLOGICAL MONITORING METHOD**

(30) Priority: 22.12.2023 TW 112150251
(71) Applicant: Giga-Byte Technology Co., Ltd., New Taipei City 231 (TW)
(72) Inventor: CHEN, Hung-Cheng, 231 New Taipei City (TW); LIN, Shu Jen, 231 New Taipei City (TW); CHEN, Yi Tse, 231 New Taipei City (TW); LIN, Chiang Lung, 231 New Taipei City (TW)
(74) Representative: Becker, Eberhard

(57) **Abstract**

Electronic system (1) and physiological monitoring method are provided. The electronic system (1) includes a camera (10), an auxiliary feature sensor (11), and an accelerator (12). The camera (10) is configured to take a facial image of a user. The auxiliary feature sensor (11) is configured to sense an auxiliary feature of the user. The accelerator (12) is configured to calculate an emotional status of the user according to the facial image. When it is determined that the emotional status meets a preset emotional status, the accelerator (12) is configured to calculate an emotional level of the user by analyzing the auxiliary feature, and determine whether to issue an alert signal accordingly.

## Description

### BACKGROUND

### Technical Field

The disclosure relates to an electronic system and a physiological monitoring method, and in particular relates to an electronic system and a physiological monitoring method configured to monitor a health status.

### Description of Related Art

In contemporary society, people often find themselves under conditions of high pressure and long working hours. In this situation, how to appropriately remind users so that they may make immediate responses or improvements is one of the most important issues to address.

### SUMMARY

An electronic system and a physiological monitoring method, which may monitor a physiological status of a user and issue alerts when necessary, are provided in the disclosure.

The electronic system of the disclosure includes a camera, an auxiliary feature sensor, and an accelerator. The camera is configured to take a facial image of a user. The auxiliary feature sensor is configured to sense an auxiliary feature of the user. The accelerator is configured to calculate an emotional status of the user according to the facial image. When it is determined that the emotional status meets a preset emotional status, the accelerator is configured to determine an emotional level of the user by analyzing the auxiliary feature, and determine whether to issue an alert signal accordingly.

The physiological monitoring method of the disclosure includes the following operation. A facial image of a user is taken. An auxiliary feature of the user is sensed. An emotional status of the user is calculated according to the facial image. When it is determined that the emotional status meets a preset emotional status, an emotional level of the user is determined by analyzing the auxiliary feature, and whether to issue an alert signal is determined accordingly.

Based on the above, the electronic system and physiological monitoring method of the disclosure may continuously monitor the emotional and physiological status of the user, and determine whether to issue an alert signal accordingly.

### BRIEF DESCRIPTION OF THE DRAWINGS

FIG. 1 illustrates a block schematic diagram of an electronic system 1 according to an embodiment of the disclosure.
FIG. 2 illustrates a flowchart of a physiological monitoring method according to an embodiment of the disclosure.
FIG. 3 is a flowchart of a physiological monitoring method according to an embodiment of the disclosure.

### DETAILED DESCRIPTION OF DISCLOSED EMBODIMENTS

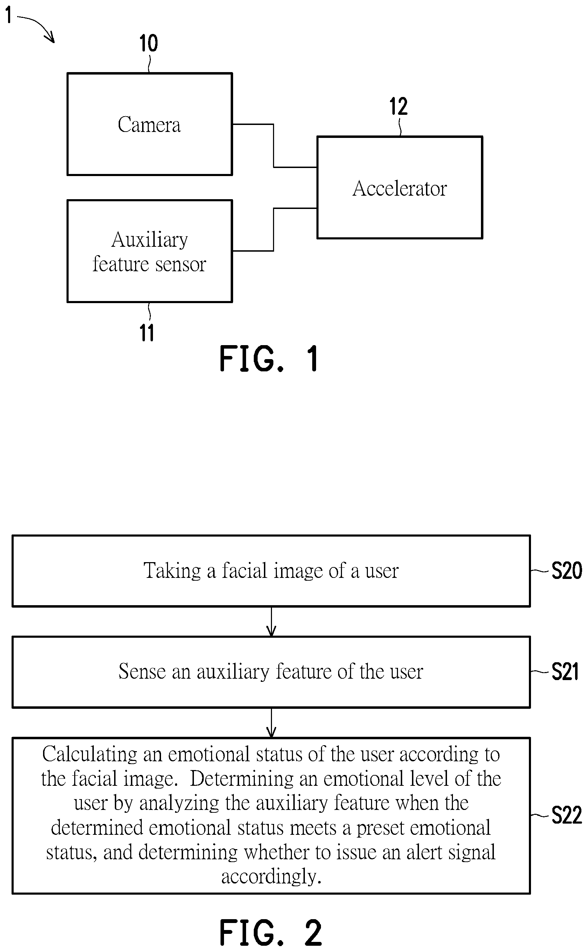

FIG. 1 illustrates a block schematic diagram of an electronic system 1 according to an embodiment of the disclosure. As shown in FIG. 1, the electronic system 1 includes a camera 10, an auxiliary feature sensor 11, and an accelerator 12. The camera 10 may be configured to take a facial image of a user. The auxiliary feature sensor 11 may be configured to sense an auxiliary feature of the same user. The accelerator 12 is coupled to the camera 10 and the auxiliary feature sensor 11. The accelerator 12 may be configured to calculate an emotional status of the user according to the facial image. When it is determined that the emotional status meets a preset emotional status, an emotional level of the user is determined by analyzing the auxiliary feature, and whether to issue an alert signal is determined accordingly.

To put it simply, in some embodiments, the accelerator 12 of the electronic system 1 may first determine the emotional status of the user according to the facial image, then assist in determining the emotional level of the user according to the auxiliary feature, and determine whether to issue an alert signal according to the emotional level in the emotional status.

FIG. 2 illustrates a flowchart of a physiological monitoring method according to an embodiment of the disclosure. The physiological monitoring method shown in FIG. 2 may be applied to and executed by the electronic system 1 of FIG. 1. Specifically, the physiological monitoring method in FIG. 2 includes steps S20 to S22.

In step S20, the electronic system 1 may take a facial image of the user through the camera 10 and provide it to the accelerator 12. In step S21, the auxiliary feature sensor 11 may sense the auxiliary feature of the user. Finally, in step S22, the accelerator 12 of the electronic system 1 may calculate an emotional status of the user according to the facial image. When it is determined that the emotional status meets a preset emotional status, an emotional level of the user is determined by analyzing the auxiliary feature, and whether to issue an alert signal is determined accordingly.

Specifically, in step S22, after receiving the facial image, the accelerator 12 may determine the emotional status of the user through image recognition. In some embodiments, the accelerator 12 may pre-store at least one image feature of a preset emotional status, and determine whether the facial image of the user belongs to a specific preset emotional status by identifying whether the facial image meets any image feature. For example, in order to monitor the health status of the user, the image feature stored by the accelerator 12 may be, for example, excitement, fatigue, anxiety, anger, etc., or other more agitated emotional statuses. The accelerator 12 may determine whether the emotion of the user falls into one of the more agitated emotional statuses by recognizing the facial image.

Furthermore, when the accelerator 12 determines that the user falls into a preset emotional status, the accelerator 12 may further obtain the auxiliary feature sensed by the auxiliary feature sensor 11 to determine the current emotional level of the user in that emotional status. In some embodiments, the auxiliary feature sensor 11 may be, for example, an infrared camera, configured to sense an auxiliary feature such as a facial temperature change signal. Alternatively, the auxiliary feature sensor 11 may be, for example, other wearable or non-wearable sensing devices, which may be configured to sense auxiliary features such as at least one of blood pressure, blood oxygen, pulse, body temperature, or other similar parameters.

Taking the facial temperature change as an example, when the accelerator 12 determines that the user is likely to be in an excited status according to the facial image, the accelerator 12 may further refer to the facial temperature change signal to determine whether the facial temperature change of the user is too high, and further determine whether the emotional level of the user under the excitement emotion is too high, that is, an overly excited emotional status. Specifically, the accelerator 12 may sense temperature changes in some or all areas of the face of the user, such as the forehead, cheeks, etc., or sense the average temperature change of the entire face. In some embodiments, when the accelerator 12 determines that the emotional status of the user changes to a more agitated emotional status such as excitement or anger according to the facial image, the user generally has a higher facial temperature, therefore the accelerator 12 may determine whether the rising change of the facial temperature of the user is still within the normal range through the facial temperature change signal. On the contrary, when the accelerator 12 determines that the emotional status of the user changes to a more negative emotional status such as depression or fatigue, the user generally has a lower facial temperature, and the accelerator 12 may determine whether the decreasing change of the facial temperature of the user is within the normal range through the facial temperature change signal.

In some embodiments, the normal range of the facial temperature change signal may have a fixed or variable temperature threshold. For example, the variable temperature threshold may take into account changes in the reference room temperature or other variables. For example, the accelerator 12 may also refer to the rise or fall of the room temperature change. After deducting the change in facial temperature change due to the room temperature change, the emotional level is determined according to the deducted facial temperature change, thereby increasing the accuracy of determining the emotional level. In this way, the accelerator 12 may determine the emotional status of the user according to the facial image, and determine the emotional level of the user according to the auxiliary feature.

In some embodiments, the auxiliary feature used by the accelerator 12 to determine the emotion level may also be one of physiological change signals such as blood pressure, blood oxygen, pulse, body temperature, etc. Specifically, similar to the above-mentioned process of determining the emotional level according to the facial temperature change, the accelerator 12 may determine the emotional level of the user according to whether the physiological change signal exceeds a preset range. For example, the normal range of a pulse rate of the user should fall between 60 and 100 beats per minute. The accelerator 12 may determine the emotional level of the user by determining whether the pulse of the user exceeds the normal range or whether the pulse change quantity increases or decreases excessively.

Then, when the accelerator 12 determines that the emotion of the user is too high or too low according to the auxiliary feature, the accelerator 12 may determine and issue an alert signal accordingly. In some embodiments, the accelerator 12 can, for example, remind the user in the form of image display, sound, vibration, etc. Alternatively, the alert signal may be sent by the electronic system 1 to the emergency contact of the user through a network. In this way, the electronic system 1 may determine whether the physiological status of the user is detrimental to health according to the calculated emotional status and the corresponding emotional level, and may issue a corresponding alert signal according to the urgency level.

In some embodiments, the disposition location of the accelerator 12 may be, for example, integrated on the motherboard, or separately disposed on a keyboard, mouse, connector, or peripheral device. In some embodiments, the accelerator 12 may be, for example, a central processing unit (CPU), a graphics processing unit (GPU), an arithmetic logic unit (ALU), a field programmable gate array (FPGA), any other type of integrated circuit, state machine, an advanced RISC machine (ARM) based processor, other similar elements, or a combination of the above elements. The accelerator 12 may store a pre-trained artificial intelligence (Al) model. The artificial intelligence model may be trained by a data set including multiple facial images with pre-labeled emotional statuses, so that the artificial intelligence model may classify subsequent input facial images to determine whether the facial images fall into any of the preset emotional statuses. The accelerator 12 may perform image recognition on the facial image by executing the artificial intelligence model to determine the emotional status of the user. For example, the artificial intelligence model stored in the accelerator 12 may be a machine learning model of supervised or unsupervised learning, or a deep learning model under the above classification, such as a region-based convolutional neural network (RCNN) series, which may be, for example, RCNN, fast RCNN, mask RCNN and faster RCNN, SSD and YOLO (you only look once) series (e.g., YOLO, YOLOv2, YOLO 9000 and YOLOv3) and other models. Furthermore, the above-mentioned artificial intelligence model may be built based on decision trees, logistic regression, SVM (support vector machine), naive Bayes, kNN, or other suitable algorithms. In addition, for the aforementioned deep learning model, one may also consider aspects such as its image preprocessing, model architecture, model depth, convolution layer parameters, pooling layer, activation function, loss function, specific architecture of each model, design philosophies, accuracy, and execution speed. By combining, adding, adjusting, and modifying these aspects, one may create a customized deep learning model.

In some embodiments, the auxiliary feature sensor 11 may have one or more sensors according to the requirements of the electronic system 1. For example, when the accelerator 12 needs to evaluate the emotional level based on the facial temperature change signal, the auxiliary feature sensor 11 includes, for example, an infrared camera to take and display the temperature change of the face of the user. When the accelerator 12 determines the emotional level according to, for example, blood pressure, blood oxygen, pulse, body temperature or other similar physiological change signals, the auxiliary feature sensor 11 may be, for example, a wearable sensing device such as a watch or bracelet, or may be a non-wearable sensing device disposed on a mouse, mouse pad, or earphones.

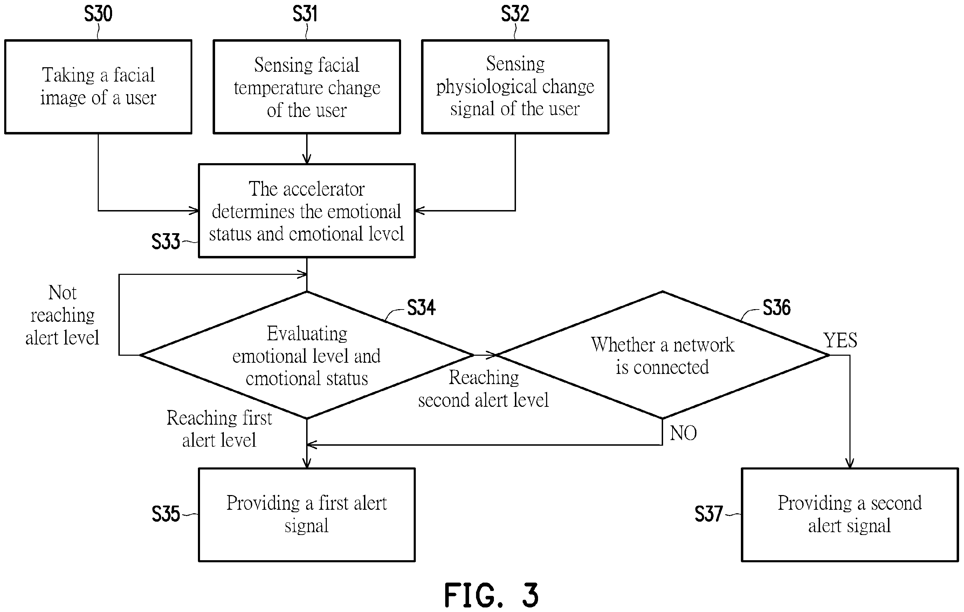

FIG. 3 is a flowchart of a physiological monitoring method according to an embodiment of the disclosure. The physiological monitoring method shown in FIG. 3 may be applied to and executed by the electronic system 1 of FIG. 1. Specifically, the physiological monitoring method in FIG. 3 includes steps S30 to S37.

In step S30, the camera 10 may take a facial image of the user and provide it to the accelerator 12. In step S31, the auxiliary feature sensor 11 may be, for example, an infrared temperature sensor, which may be configured to sense the facial temperature of the user, and provide a facial temperature change signal to the accelerator 12 accordingly. In step S32, in addition to the above-mentioned infrared temperature sensor, the auxiliary feature sensor 11 may also have other wearable or non-wearable sensing instruments for sensing physiological change signals of at least one of parameters such as blood pressure, blood oxygen, pulse, body temperature, or other similar parameters of the user, and provide them to the accelerator 12.

In step S33, the accelerator 12 may determine the emotional status of the user according to the facial image. For example, the accelerator 12 may identify the lines in the facial image through image recognition to determine the emotional status of the user. It may discern whether the user is in a state of excitement, fatigue, anxiety, anger, or other more agitated emotional statuses, or in a state of depression, fatigue, or other more negative emotional statuses. In these emotional statuses, the accelerator 12 may further determine the emotional level of the user in the emotion according to the facial temperature change signal and the physiological change signal.

In step S34, the accelerator 12 evaluates whether the emotional status and emotional level of the user reach a first alert level or a second alert level, and accordingly determines whether to proceed to step S35 to issue the first alert signal, or to proceed to steps S36 to S37 to issue the second alert signal, or, in the absence of reaching any alert level, to continuously monitor the emotional status of the user, the facial temperature change, and the physiological condition. Specifically, the accelerator 12 may determine the emotional status of the user according to the facial image, and determine the emotional level of the user in the emotional status of the user according to the facial temperature change signal. When the accelerator 12 determines that the emotional level of the user falls into the first range, which may include when the emotion level is higher (e.g., higher than the first upper limit) or lower (e.g., lower than the first lower limit), the accelerator 12 may determine that the first alert level has been reached and proceed to step S35 to instruct the first alert signal to be issued.

Furthermore, the accelerator 12 may also use the physiological change signal as an auxiliary to the facial temperature change signal to further confirm whether the physiological status of the user has reached the more urgent second alert status. For example, when the accelerator 12 determines that the emotional level of the user falls into the second range according to the facial temperature change signal, which may include when the emotion level is too high (e.g., higher than the second upper limit, and the second upper limit is greater than the first upper limit) or the emotion level is too low (e.g., lower than the second lower limit, and the second lower limit is less than the first lower limit), the accelerator 12 may further refer to whether the physiological change signal exceeds a preset range. When the accelerator 12 determines that the emotion level of the user is too high or too low, and the physiological change signal exceeds the preset range, the accelerator 12 may determine that the emotion of the user has reached the second alert level accordingly, which means that the physiological status of the user has reached a more urgent situation, and accordingly proceed to step S36 to attempt to issue a second alert signal.

In step S36, the electronic system 1 may evaluate whether its network function is turned on or whether it has the permission to turn on the network function, and when the network function is turned on or it has the permission to enable the network function, the electronic system 1 proceeds to step S37 to issue a second alert signal to the emergency contact of the user through the network. When the evaluation result is negative, the accelerator 12 gives up issuing the second alert signal.

In summary, the electronic system and physiological monitoring method of the disclosure may perform real-time monitoring of the physiological status of the user to issue an alert signal when the physiological status of the user is critical, and effectively issue alerts for the critical status of the user caused by fatigue, excessive use of electronic devices or stress, so that the user may take emergency measures accordingly.

## Claims

1. An electronic system (1), comprising:
a camera (10), configured to take a facial image of a user;
an auxiliary feature sensor (11), configured to sense an auxiliary feature of the user; and
an accelerator (12), configured to calculate an emotional status of the user according to the facial image, determine an emotional level of the user in the emotional status by analyzing the auxiliary feature when it is determined that the emotional status meets a preset emotional status, and determine whether to issue an alert signal accordingly.

2. The electronic system (1) according to claim 1, wherein the accelerator (12) determines the emotional status of the user by recognizing the facial image through an image recognition technology.

3. The electronic system (1) according to claim 2, wherein the accelerator (12) determines the emotional status of the user by performing the image recognition technology on the facial image through executing an artificial intelligence model.

4. The electronic system (1) according to claim 1, wherein the auxiliary feature comprises a facial temperature change signal, and the accelerator (12) determines the emotional level of the user in the emotional status by analyzing the facial temperature change signal of the user.

5. The electronic system (1) according to claim 1, wherein the auxiliary feature comprises a physiological change signal, the physiological change signal comprises at least one of blood pressure, blood oxygen, pulse, and body temperature, the accelerator (12) determines the emotional level of the user in the emotional status by analyzing the physiological change signal.

6. The electronic system (1) according to claim 4, wherein the auxiliary feature further comprises a physiological change signal, the accelerator (12) determines the emotional level of the user by analyzing the facial temperature change signal, and then verifies the emotional level by analyzing the physiological change signal.

7. The electronic system (1) according to claim 1, wherein the accelerator (12) instructs a first alert device to issue a first alert signal when the accelerator (12) determines that the emotion level of the user falls into a first range,
wherein the accelerator (12) issues a second alert signal through a network after confirming that a network setting of the electronic system (1) is turned on when the accelerator (12) determines that the emotion level of the user falls into a second range.

8. The electronic system (1) according to claim 1, wherein the accelerator (12) is disposed on a motherboard or a connector, or is disposed on an edge device connected through a connection terminal or a transmission line.

9. A physiological monitoring method, comprising:
taking a facial image of a user;
sensing an auxiliary feature of the user; and
calculating an emotional status of the user according to the facial image, determining an emotional level of the user in the emotional status by analyzing the auxiliary feature when it is determined that the emotional status meets a preset emotional status, and determining whether to issue an alert signal accordingly.

10. The physiological monitoring method according to claim 9, comprising:
determining the emotional status of the user by recognizing the facial image through an image recognition technology.

11. The physiological monitoring method according to claim 10, comprising:
determining the emotional status of the user by performing the image recognition technology on the facial image through executing an artificial intelligence model.

12. The physiological monitoring method according to claim 9, wherein the auxiliary feature comprises a facial temperature change signal, the physiological monitoring method comprises:
determining the emotional level of the user in the emotional status by analyzing the facial temperature change signal of the user.

13. The physiological monitoring method according to claim 9, wherein the auxiliary feature comprises a physiological change signal, the physiological change signal comprises at least one of blood pressure, blood oxygen, pulse, and body temperature, the physiological monitoring method comprises:
determining the emotional level of the user in the emotional status by analyzing the physiological change signal.

14. The physiological monitoring method according to claim 12, wherein the auxiliary feature further comprises a physiological change signal, the physiological monitoring method comprises:
determining the emotional level of the user by analyzing the facial temperature change signal, and then verifying the emotional level by analyzing the physiological change signal.

15. The physiological monitoring method according to claim 9, comprising:
instructing a first alert signal to be issued when determining that the emotion level of the user falls into a first range,
issuing a second alert signal through a network after confirming that a network setting is turned on when determining that the emotion level of the user falls into a second range.

## Amended claims

### Amended claims in accordance with Rule 137(2) EPC.

1. An electronic system (1), **characterized by** comprising:
a camera (10), configured to take a facial image of a user;
an auxiliary feature sensor (11), configured to sense an auxiliary feature of the user; and
an accelerator (12), configured to calculate an emotional status of the user according to the facial image, determine an emotional level of the user in the emotional status by analyzing the auxiliary feature according to the emotional status when it is determined that the emotional status meets a preset emotional status, and determine whether to issue an alert signal accordingly.

2. The electronic system (1) according to claim 1, wherein the accelerator (12) determines the emotional status of the user by recognizing the facial image through an image recognition technology.

3. The electronic system (1) according to claim 2, wherein the accelerator (12) determines the emotional status of the user by performing the image recognition technology on the facial image through executing an artificial intelligence model.

4. The electronic system (1) according to claim 1, wherein the auxiliary feature comprises a facial temperature change signal, and the accelerator (12) determines the emotional level of the user in the emotional status by analyzing the facial temperature change signal of the user.

5. The electronic system (1) according to claim 1, wherein the auxiliary feature comprises a physiological change signal, the physiological change signal comprises at least one of blood pressure, blood oxygen, pulse, and body temperature, the accelerator (12) determines the emotional level of the user in the emotional status by analyzing the physiological change signal.

6. The electronic system (1) according to claim 4, wherein the auxiliary feature further comprises a physiological change signal, the accelerator (12) determines the emotional level of the user by analyzing the facial temperature change signal, and then verifies the emotional level by analyzing the physiological change signal.

7. The electronic system (1) according to claim 1, wherein the accelerator (12) instructs a first alert device to issue a first alert signal when the accelerator (12) determines that the emotion level of the user falls into a first range,
wherein the accelerator (12) issues a second alert signal through a network after confirming that a network setting of the electronic system (1) is turned on when the accelerator (12) determines that the emotion level of the user falls into a second range.

8. The electronic system (1) according to claim 1, wherein the accelerator (12) is disposed on a motherboard or a connector, or is disposed on an edge device connected through a connection terminal or a transmission line.

9. A physiological monitoring method, **characterized by** comprising:
taking a facial image of a user;
sensing an auxiliary feature of the user; and
calculating an emotional status of the user according to the facial image, determining an emotional level of the user in the emotional status by analyzing the auxiliary feature according to the emotional status when it is determined that the emotional status meets a preset emotional status, and determining whether to issue an alert signal accordingly.

10. The physiological monitoring method according to claim 9, comprising:
determining the emotional status of the user by recognizing the facial image through an image recognition technology.

11. The physiological monitoring method according to claim 10, comprising:
determining the emotional status of the user by performing the image recognition technology on the facial image through executing an artificial intelligence model.

12. The physiological monitoring method according to claim 9, wherein the auxiliary feature comprises a facial temperature change signal, the physiological monitoring method comprises:
determining the emotional level of the user in the emotional status by analyzing the facial temperature change signal of the user.

13. The physiological monitoring method according to claim 9, wherein the auxiliary feature comprises a physiological change signal, the physiological change signal comprises at least one of blood pressure, blood oxygen, pulse, and body temperature, the physiological monitoring method comprises:
determining the emotional level of the user in the emotional status by analyzing the physiological change signal.

14. The physiological monitoring method according to claim 12, wherein the auxiliary feature further comprises a physiological change signal, the physiological monitoring method comprises:
determining the emotional level of the user by analyzing the facial temperature change signal, and then verifying the emotional level by analyzing the physiological change signal.

15. The physiological monitoring method according to claim 9, comprising:
instructing a first alert signal to be issued when determining that the emotion level of the user falls into a first range,
issuing a second alert signal through a network after confirming that a network setting is turned on when determining that the emotion level of the user falls into a second range.
